# EUROPÄISCHE PATENTSCHRIFT

(11) **EP 0 885 606 B1**
(45) Veröffentlichungstag und Bekanntmachung des Hinweises auf die Patenterteilung: **30.07.2003**
(21) Anmeldenummer: 98250188.4
(22) Anmeldetag: 28.05.1998
(51) Int. Cl.: A61K 6/06, A61K 6/033, C03C 3/091, C03C 3/093, C03C 3/097, C03C 3/118, C03C 10/16, C03C 8/06, C03C 8/08

(54) **Alkali-Silicat-Glas**
Alkali-silicate glass
Verre de silicate d'alcali

(30) Priorität: 12.06.1997 DE 19725552
(43) Veröffentlichungstag der Anmeldung: 23.12.1998
(73) Patentinhaber: Ivoclar Vivadent AG, 9494 Schaan (LI)
(72) Erfinder: Drescher, Helga, 6800 Feldkirch (AT); Frank, Martin, Dipl.-Ing., 9494 Schaan (LI); Höland, Wolfram, Prof. Dr., 9494 Schaan (LI); Rheinberger, Volker, Dr., 9490 Vaduz (LI)
(74) Vertreter: UEXKÜLL & STOLBERG

(56) Entgegenhaltungen:
- EP-A- 0 695 726
- EP-A- 0 827 941
- DE-A- 4 020 893
- US-A- 4 772 436
- PATENT ABSTRACTS OF JAPAN vol. 013, no. 523 (C-657), 21. November 1989 -& JP 01 212248 A (NIPPON ELECTRIC GLASS CO LTD), 25. August 1989

## Beschreibung

Die Erfindung betrifft Alkali-Silicat-Glas und insbesondere solches, das sich zur gewünschten Einstellung der optischen Eigenschaften und der Verarbeitungseigenschaften von Beschichtungs- und Verblendmaterial für keramische Dentalrestaurationen eignet.

Neben metallischen Dentalrestaurationen, die aus ästhetischen Gründen mit keramischen Schichten verblendet werden, finden in der Zahnheilkunde in zunehmendem Maße vollkeramische Restaurationen Verwendung, bei denen auf einen Kern aus keramischem Werkstoff auch ein keramisches Verblend- oder Beschichtungsmaterial aufgebracht wird. Für den Einsatz als Kern- und auch als Beschichtungsmaterial kommen u.a. Glaskeramiken in Frage.

Vor allem die optischen Eigenschaften sowie die Verarbeitungseigenschaften von glaskeramischem Beschichtungsmaterial sind jedoch vielfach nicht zufriedenstellend. So zeigen die eingesetzten Glaskeramiken infolge ihres hohen Kristallgehaltes eine starke Trübung, die gerade bei Dentalrestaurationen für den Schneidezahnbereich nicht annehmbar ist. Darüber hinaus haben die Glaskeramiken in vielen Fällen einen sehr hohen Ausdehnungskoeffizienten, weshalb sie als Beschichtungsmaterial für Kerne aus Glaskeramik mit niedrigem Ausdehnungskoeffizienten, wie z.B. Lithiumdisilicat-Glaskeramik, ungeeignet sind. Infolge der mangelhaften Anpassung der Ausdehnungskoeffizienten kann es zu unerwünschten Ablösungen des Beschichtungsmaterials kommen.

Weiter ist es bekannt, daß gerade leucithaltige Glaskeramiken sehr hohe lineare thermische Ausdehnungskoeffizienten besitzen. Diese sind auf den Gehalt an Leucit-Kristallen zurückzuführen, die durch gesteuerte Kristallisation eines entsprechenden Ausgangsglas gebildet werden.

Aus der EP-A-695 726 sind Alkali-Zink-Silicat-Gläser bekannt, die zum Verblenden von vornehmlich metallischen Dentalsuprastrukturen geeignet sind und kein B₂O₃ enthalten. Die Gläser bilden jedoch bei Wärmebehandlung im Bereich 600 °C bis 1000 °C und damit für die zahntechnische Weiterverarbeitung üblichen Bedingungen entsprechende Glaskeramiken, die infolge ihres Kristallgehaltes stark getrübt sind und sich daher nicht zur Einstellung einer hohen Transluzenz bei einem glaskeramischen Beschichtungsmaterial eignen. Der Gehalt an Kristallen, insbesondere Leucit, führt außerdem zu unerwünscht hohen Ausdehnungskoeffizienten und Sintertemperaturen, so daß sie für die Verblendung von keramischen Substraten mit niedrigen Ausdehnungskoeffizienten nicht zufriedenstellend sind.

Der Erfindung liegt nunmehr die Aufgabe zugrunde, ein Glas zur Verfügung zu stellen, welches bei den üblichen Bedingungen einer zahntechnischen Verarbeitung im Temperaturbereich von 600 °C bis 1000 °C nicht kristallisiert, einen niedrigen Ausdehnungskoeffizienten, eine niedrige Sintertemperatur, eine hohe chemische Stabilität sowie eine hohe Transluzenz aufweist und demgemäß insbesondere dentalem glaskeramischen Beschichtungsmaterial zugesetzt werden kann, um dessen Eigenschaften zu verbessern.

Diese Aufgabe wird durch das Alkali-Silicat-Glas nach den Ansprüchen 1 bis 6 gelöst.

Gegenstand der vorliegenden Erfindung sind ebenfalls das Dentalmaterial nach den Ansprüchen 7 bis 12, die Verwendung nach den Ansprüchen 13 bis 16 sowie das geformte Dentalprodukt nach den Ansprüchen 17 bis 20.

Das erfindungsgemäße Alkali-Silicat-Glas ist dadurch gekennzeichnet, daß es die folgenden Komponenten enthält:

| Komponente | Gew.-% |
|---|---|
| SiO₂ | 55,0 bis 71,0 |
| Al₂O₃ | 5,0 bis 16,0 |
| B₂O₃ | 0,2 bis 10,0 |
| K₂O | 4,5 bis 10,0 |
| Na₂O | 3,0 bis 14,0. |
| CaO | 0,5 bis 3,0 |
| F | 0,2 bis 2,0 |

SiO₂ ist vorzugsweise in einer Menge von 55,0 bis 65,0 Gew.-% vorhanden.

Das erfindungsgemäße Glas kann zusätzlich noch mindestens eine der folgenden Komponenten enthalten:

| Komponente | Gew.-% |
|---|---|
| P₂O₅ | 0 bis 0,6 |
| Li₂O | 0 bis 4,0 |
| BaO | 0 bis 5,0 |
| ZnO | 0 bis 4,0 |
| TiO₂ + ZrO₂ | 0,2 bis 5,0 |
| CeO₂ | 0 bis 2,0. |

Mit Ausnahme von TiO₂ und ZrO₂ ist die untere Grenze dieser zusätzlichen Komponenten üblicherweise 0,05 Gew.-%.

Für die einzelnen Komponenten des erfindungsgemäßen Alkali-Silicat-Glases existieren bevorzugte Mengenbereiche. Diese können unabhängig voneinander gewählt werden und sind wie folgt:

| Komponente | Gew.-% |
|---|---|
| SiO₂ | 60,0 bis 65,0 |
| Al₂O₃ | 6,0 bis 10,0 |
| B₂O₃ | 0,5 bis 8,1 |
| K₂O | 5,5 bis 9,0 |
| Na₂O | 3,5 bis 10,0 |

Besonders bevorzugte Mengenbereiche für die einzelnen Komponenten des erfindungsgemäßen Glases sind wie folgt, und diese können unabhängig voneinander gewählt werden:

| Komponente | Gew.-% |
|---|---|
| SiO₂ | 61,0 bis 64,0 |
| Al₂O₃ | 7,0 bis 9,0 |
| B₂O₃ | 0,5 bis 4,0 |
| Na₂O | 7,0 bis 9,0 |
| CaO | 0,5 bis 1,5 |
| F | 1,0 bis 2,0 |
| Li₂O | 0 bis 3,0 |
| BaO | 1,5 bis 3,5 |
| ZnO | 2,0 bis 3,5. |

Alle vorstehenden Mengenangaben in Gew.-% beziehen sich auf das Glas.

Zur Herstellung des erfindungsgemäßen Glases wird vorzugsweise so vorgegangen, daß geeignete Ausgangsmaterialien, wie z.B. Carbonate, Oxide und Fluoride, bei einer Temperatur im Bereich von 1350 °C bis 1650 °C, vorzugsweise 1400 °C bis 1600 °C, über einen Zeitraum von 30 Minuten bis 4 Stunden, vorzugsweise eine Stunde bis 2,5 Stunden, unter Bildung einer homogenen Schmelze erschmolzen werden. Das erschmolzene Glas wird dann üblicherweise in Wasser abgeschreckt, d.h. gefrittet, und nach Trocknen auf die gewünschte Korngröße gemahlen.

Mittels rasterelektronenmikroskopischer Untersuchungen wurde festgestellt, daß das erfindungsgemäße Glas frei von Kristallen ist. Weiter zeigte sich, daß das Glas auch die bei einer üblichen zahntechnischen Weiterverarbeitung durch Sinterung herrschenden Bedingungen übersteht, ohne daß es zu der bei bekannten Gläser auftretenden Bildung von Kristallen kommt. Selbst bei einer thermischen Behandlung im Bereich von 600 °C bis 1000 °C für 1 Minute bis 2 Stunden trat eine Kristallisation nicht auf.

Dieses Verhalten ist vermutlich auf die spezielle Zusammensetzung des erfindungsgemäßen Glases zurückzuführen.

Das erfindungsgemäße Glas weist üblicherweise eine Sintertemperatur von 650 °C bis 1150 °C auf. Besonders bevorzugt sind solche Gläser, die ein Sintertemperatur von 700 °C bis 1050 °C haben. Ganz besonders bevorzugt ist Glas, das bei niedrigen Temperaturen von 750 °C bis 880 °C zusammengesintert und damit verarbeitet werden kann.

Zur Durchführung der Sinterung wird in der Regel eine Aufheizrate von 3 °C bis 100 °C/min und bevorzugt von 30 °C bis 80 °C/min sowie eine Haltezeit bei der Sintertemperatur von 10 Sekunden bis 1 Stunde und bevorzugt 30 Sekunden bis 5 Minuten gewählt. Dabei ist es vorteilhaft, die Sinterung im Vakuum durchzuführen, damit der Sinterkörper möglichst wenig Poren aufweist.

Der lineare thermische Ausdehnungskoeffizient des erfindungsgemäßen Glases beträgt üblicherweise 5,5 bis 12,5 × 10⁻⁶K⁻¹, bevorzugt 6,0 bis 11,0 × 10⁻⁶K⁻¹, gemessen im Temperaturintervall von 100 °C bis 400 °C.

Das erfindungsgemäße Glas wird entweder alleine oder zusammen mit weiteren Komponenten bevorzugt als Dentalmaterial eingesetzt.

Hierzu wird es üblicherweise in Form eines Pulvers mit einer mittleren Teilchengröße von weniger als 90 µm verwendet. Als weitere Komponenten kommen Glaskeramiken und andere Gläser, aber auch Farbstoffe, insbesondere Farbpigmente, Oxide der 3d-Elemente oder Metallkolloide, sowie Fluoreszenzstoffe, insbesondere mit d- und f-Elementen dotiertes Ytterbium-Silicat, in Frage.

Besonders vorteilhaft ist Dentalmaterial, das als weitere Komponente mindestens eine Apatit-Glaskeramik enthält.

Dabei ist eine Apatit-Glaskeramik bevorzugt, die CaO, P₂O₅ und F in einem Mol-Verhältnis von CaO : P₂O₅ : F von 1 : 0,020 bis 1,5 : 0,03 bis 4,2 und als Hauptkristallphase Apatit-Kristalle enthält. Derartige Apatit-Glaskeramiken zeichnen sich durch eine besonders hohe chemische Stabilität aus, was gerade für den Einsatz bei dentalen Restaurationen von großer Bedeutung ist.

Weiter ist auch die Verwendung einer Apatit-Glaskeramik bevorzugt, die mindestens eine der folgenden Komponenten aufweist und als Hauptkristallphase Apatit-Kristalle enthält:

| Komponente | Gew.-% |
|---|---|
| SiO₂ | 45,0 bis 70,0 |
| Al₂O₃ | 5,0 bis 22,0 |
| P₂O₅ | 0,5 bis 6,5 |
| K₂O | 3,0 bis 8,5 |
| Na₂O | 4,0 bis 13,0 |
| CaO | 1,5 bis 11,0 |
| F | 0,1 bis 2,5. |

Besonders bevorzugt enthält diese Apatit-Glaskeramik zusätzlich mindestens eine der folgenden Komponenten:

| Komponente | Gew.-% |
|---|---|
| B₂O₃ | 0 bis 8,0 |
| La₂O₃ | 0 bis 5,0 |
| Li₂O | 0 bis 5,0 |
| BaO | 0 bis 5,0 |
| MgO | 0 bis 5,0 |
| ZnO | 0 bis 5,0 |
| SrO | 0 bis 7,0 |
| TiO₂ | 0 bis 4,0 |
| ZrO₂ | 0 bis 4,0 |
| CeO₂ | 0 bis 3,0. |

Vorstehende Angaben in Gew.-% beziehen sich auf die Apatit-Glaskeramik.

Die oben beschriebenen Apatit-Glaskeramiken werden hergestellt, indem aus geeigneten Ausgangsmaterialien, wie Oxiden, Carbonaten und Fluoriden, ein Ausgangsglas bei Temperaturen von 1200 °C bis 1650 °C erschmolzen wird, dieses in Wasser eingegossen wird und das gebildete Glasgranulat, ggf. nach weiterem Zerkleinern, einer thermischen Behandlung von mehr als 900 °C und bis zu 1200 °C für eine Dauer von 30 Minuten bis 6 Stunden unterzogen wird.

Die erhaltenen Apatit-Glaskeramiken zeichnen sich durch eine hohe Transluzenz, hohe chemische Stabilität sowie einen niedrigen Ausdehnungskoeffizienten aus. Diese Eigenschaften sind vermutlich auf ihre spezielle Zusammensetzung sowie die bei ihrer Herstellung erzeugten Apatit-Kristalle zurückzuführen, die insbesondere nadelförmige Morphologie haben und damit den Apatit-Kristallen des natürlichen Zahnmaterials ähneln.

Das erfindungsgemäße Dentalmaterial hat normalerweise einen linearen thermischen Ausdehnungskoeffizienten von 5,5 bis 12,5 x 10⁻⁶ K⁻¹, gemessen im Bereich von 100 °C bis 400 °C. Der jeweils gewünschte Koeffizient kann dabei durch geeignete Wahl der Art des Alkali-Silicat-Glases und etwaiger weiterer Komponenten, sowie deren Mengen eingestellt werden. Günstige Dentalmaterialen enthalten 10 bis 90 Gew.-% Alkali-Silicat-Glas und 90 bis 10 Gew.-% weitere Komponenten, bezogen auf das Dentalmaterial.

Das Dentalmaterial eignet sich zur Beschichtung von Substraten und insbesondere zur Beschichtung oder Verblendung von dentalen Restaurationen. Die Beschichtung erfolgt dabei insbesondere durch Aufbringen des Dentalmaterials auf das ausgewählte Substrat und anschließendes Sintern im Temperaturbereich von 650 °C bis 1150 °C.

Bevorzugt wird dabei zunächst ein Pulver des erfindungsgemäßen Glases mit einem Pulver der ggf. vorhandenen weitere Komponenten gemischt und durch Zugabe von wäßrigen Anmischlösungen zu einer Paste verarbeitet. Diese Paste wird dann auf das Substrat aufgebracht, und nach gewünschter Formgebung erfolgt das Sintern, um eine fest haftende Beschichtung oder Verblendung zu erhalten.

Das erfindungsgemäße Dentalmaterial kann als Beschichtungs- oder Verblendmaterial für Substrate, wie Dentalsuprastrukturen, z.B. auf Basis von keramischen oder glaskeramischen Werkstoffen eingesetzt werden. Aufgrund seines niedrigen Ausdehnungskoeffizienten wird es bevorzugt bei Substratwerkstoffen mit einem thermischen Ausdehungskoeffizienten von 7,0 bis 12,0, insbesondere von 8,0 bis 11,0 × 10⁻⁶K⁻¹, verwendet werden. Bevorzugt wird es zur Beschichtung oder Verblendung von ZrO₂-Keramiken, Al₂O₃-Keramiken, ZrO₂/Al₂O₃-Keramiken, keramischen oder glaskeramischen Compositwerkstoffen und Titan verwendet.

Besonders vorteilhaft wird es jedoch zur Verblendung von Substraten auf Basis von Lithiumdisilicat-Glaskeramik eingesetzt, um auf diese Weise ästhetisch sehr ansprechende vollkeramische Dentalprodukte herzustellen, die eine sehr hohe Festigkeit sowie eine ausgezeichnete chemische Stabilität haben.

Als besonders geeignet haben sich dabei Lithiumdisilicat-Glaskeramiken der folgenden Zusammensetzung erwiesen, die z.B. durch Erschmelzen entspechender Ausgangsgläser, Fritten und Wärmebehandlung bei 400 °C bis 1100 °C erhalten werden können:

| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0 |
| ZnO | 0 bis 8,0 |
| K₂O | 0 bis 13,5 |
| Li₂O | 11,0 bis 19,0 |
| P₂O₅ | 0 bis 11,0 |

wobei

| | |
|---|---|
| (a) Al₂O₃ + La₂O₃ | 0,1 bis 7,0 Gew.-% und |
| (b) MgO + ZnO | 0,1 bis 9,0 Gew.-% |

ausmachen.

Die Angaben in Gew.-% beziehen sich auf die Lithiumdisilicat-Glaskeramik.

Für die Herstellung von Beschichtungen ist erfindungsgemäßes Dentalmaterial vorteilhaft, daß einen linearen thermischen Ausdehnungskoeffizienten hat, der kleiner ist als der des zu beschichtenden Substrates. Besonders vorteilhaft ist Dentalmaterial, dessen Ausdehungskoeffizient nicht mehr als 3,0 × 10⁻⁶K⁻¹ kleiner ist als der des Substrates.

Das erfindungsgemäße Alkali-Silicat-Glas und das erfindungsgemäße Dentalmaterial können zusammen mit den ggf. vorhandenen Zusätzen zu geformten Dentalprodukten in üblicher Weise verarbeitet werden. Als erfindungsgemäße geformte Dentalprodukte, die einen Gehalt an dem Alkali-Silicat-Glas oder dem Dentalmaterial aufweisen, kommen insbesondere Dentalrestaurationen, wie z.B. ein Inlay, ein Onlay, eine Brücke, ein Stiftaufbau, eine Verblendung, eine Schale, ein Veneer, eine Facette, eine Füllung oder ein Verbinder in Frage. Besonders bevorzugte Dentalrestaurationen sind Brücken, Kronen und Teilkronen.

Die Dentalprodukte haben bevorzugt einen Kern auf Basis von keramischem oder glaskeramischem Werkstoff, insbesondere Lithiumdisilicat-Glaskeramik, auf den das erfindungsgemäße Glas oder das erfindungsgemäße Dentalmaterial aufgebracht ist.

Bevorzugte Lithiumdisilicat-Glaskeramiken sind oben bereits beschrieben worden.

Im Gegensatz zu konventionellem Glas kommt es bei dem erfindungsgemäßen Glas unter den bei seiner Sinterung herrschenden Bedingungen nicht zu einer Kristallisation, die seine Transluzenz unerwünscht erniedrigen würde. Damit gibt es im wesentlichen die Färbung des beschichteten Substrates wieder, was gerade bei der Herstellung von vollkeramischen Dentalrestaurationen von besonderem Vorteil ist.

Das Ausbleiben der Kristallbildung und insbesondere der bei bekannten Gläsern festgestellten Bildung von Leucit-Kristallen ist von besonderem Vorteil, da der hohe Ausdehnungskoeffizient von Leucit dem Glas einen hohen thermischen Ausdehungskoeffizienten verleihen würde. Damit wäre das Glas aber für die Beschichtung von Substraten mit niedrigen Ausdehnungskoeffizienten, wie z.B. ZrO₂ oder Lithiumdisilicat-Glaskeramik, nicht geeignet. Die fehlende Anpassung der Ausdehnungskoeffizienten würde zu hohen Spannungen führen, die sich regelmäßig in Rissen und Abplatzungen äußern. Diese Nachteile zeigt das erfindungsgemäße Glas infolge seines niedrigen Ausdehnungskoeffizienten nicht, so daß es sich sehr gut zur Beschichtung von Substraten mit niedrigen Ausdehnungskoeffizienten eignet.

Überdies zeigt das Glas trotz seines B₂O₃-Gehaltes eine ausgezeichnete chemische Stabilität, die für seine Verwendung als Dentalmaterial unerläßlich ist, welches in der Mundhöhle permanent von sauren Flüssigkeiten umspült wird.

Schließlich kann das Glas schon bei niedrigen Temperaturen innerhalb einer kurzen Sinterzeit auf ein Substrat aufgesintert werden, um auf diese Weise eine fest haftende Beschichtung oder Verblendung herzustellen.

Die Zumischung des Glases gerade zu Apatit-Glaskeramiken führt zu Dentalmaterialien, die gegenüber der reinen Apatit-Glaskeramik eine erhöhte Transluzenz, kürzere Sinterzeit und -temperatur sowie einen erniedrigten thermischen Ausdehnungskoeffizienten haben.

Die Erfindung wird nachstehend anhand von Beispielen näher erläutert.

### Beispiele

### Beispiele 1 bis 6

Es wurden insgesamt 6 verschiedene erfindungsgemäße Gläser mit den in Tabelle I angegebenen chemischen Zusammensetzungen hergestellt.

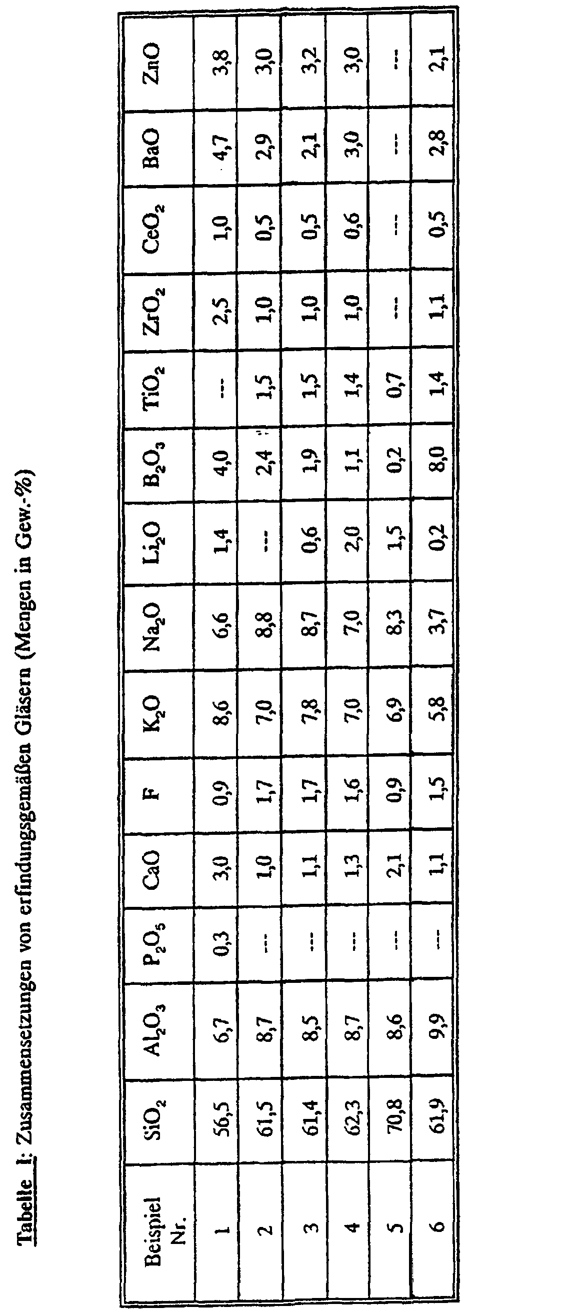

Zu ihrer Herstellung wurde jeweils ein entsprechendes Gemenge von geeigneten Oxiden, Carbonaten und Fluoriden in einem Platin/ Rhodium-Tiegel bei einer Temperatur von 1550 °C bis 1600 °C während einer Homogenisierungszeit von 1 bis 1,5 Stunden erschmolzen. Die Glasschmelze wurde in Wasser abgeschreckt, und das gebildete Granulat des Ausgangsglases wurde getrocknet und auf eine mittlere Korngröße von weniger als 90 µm aufgemahlen.

Für die hergestellten Gläser sind in Tabelle II ausgewählte Eigenschaften angegeben, die an Probekörpern aus dem jeweiligen Glas bestimmt worden sind. Die Beispiele verdeutlichen wie durch Veränderung der chemischen Zusammensetzung Gläser mit unterschiedlichen Eigenschaften erhalten werden können.

**Tabelle II**

| Beispiele | Brenntemperatur* [°C] | Tg [°C] | α-Wert ×10⁻⁸K⁻¹ (100 °C - 400 °C) | Optik | Säurebeständigkeit [µg/cm²] |
|---|---|---|---|---|---|
| 1 | 760 | 500 | 9,6 | transluzent | 26 |
| 2 | 810 | 522 | 9,1 | sehr transluzent | 17,2 |
| 3 | 770 | 494 | 9,4 | sehr transluzent | 26,3 |
| 4 | 750 | 468 | 9,4 | sehr transluzent | 17,9 |
| 6 | 880 | 543 | 6,6 | sehr transluzent | < 100 |

| | | | | | |
|---|---|---|---|---|---|
| ^{*}Brenntemperatur = Temperatur, die zur Prüfkörperherstellung durch Sinterung auf Quarz verwendet wurde (Vakuum, Haltezeit 1 Minute) | | | | | |

### Bestimmung des Ausdehnungskoeffizienten α

Zur Messung des linearen thermischen Ausdehnungskoeffizienten α wurde aus Pulver des jeweiligen Glases ein stäbchenförmiger Grünkörper hergestellt, der in einem Vakuumbrennofen mit einer Aufheizrate von 60 °C/min und einer Haltezeit von 1 Minute bei der jeweiligen Brenntemperatur gesintert wurde. Anschließend wurde ein Glanzbrand ohne Vakuum bei einer um 20 °C höheren Endtemperatur und einer Haltezeit von 1 Minute durchgeführt. An dem erhaltenen Probekörper wurde der lineare thermische Ausdehnungskoeffizient bestimmt.

### Bestimmung der Säurebeständigkeit

Die Säurebeständigkeit ist ein Maß für die chemische Stabilität gerade von im Dentalbereich eingesetzten Gläsern und Glaskeramiken, da diese in der Mundhöhle permanent der Einwirkung von sauren Substanzen ausgesetzt sind.

Die Säurebeständigkeit wurde gemäß der ISO-Vorschrift 6872:1995 bestimmt. Dazu wurden zunächst Probeplättchen mit einem Durchmesser von 12 mm und einer Dicke von 1 mm durch Zusammensintern von Glas-Granulat mit einer mittleren Teilchengröße von 90 µm hergestellt. Das Granulat wurde 1 Minute lang auf der Sintertemperatur gehalten. Dann wurden die Probeplättchen 16 Stunden lang in einer Soxhlet-Apparatur mit 4 Vol.-%iger wäßriger Essigsäure behandelt, und es wurde schließlich der eingetretene Masseverlust als Maß für die Säurebeständigkeit bestimmt.

### Beispiel 7

Dieses Beispiel beschreibt den Einsatz erfindungsgemäßer Gläser nach Beispiel 2 und 3 zusammen mit einer Apatit-Glaskeramik (A) als Beschichtungsmaterial für keramische Suprastrukturen und somit zur Herstellung von vollkeramischen Dentalprodukten.

Die Apatit-Glaskeramik (A) hatte die Zusammensetzung SiO₂ 55,5 Gew.-%, Al₂O₃ 19,2 Gew.-%, P₂O₅ 1,2 Gew.-%, CaO 2,7 Gew.-%, F 0,6 Gew.-%, K₂O 6,7 Gew.-%, Na₂O 9,7 Gew.-%, B₂O₃ 0,3 Gew.-%, TiO₂ 1,4 Gew.-%, ZrO₂ 2,2 Gew.-% und CeO₂ 0,5 Gew.-%. Zu ihrer Herstellung wurde ein Ausgangsglas entsprechender Zusammensetzung erschmolzen, gefrittet und zu einem Pulver aufgemahlen. Dieses Pulver wurde dann 1 Stunde lang bei 1020 °C wärmebehandelt. Die in der gebildeten Glaskeramik vorhandenen Kristalle konnten mittels Röntgendiffraktometrie als nadelförmige Apatit-Kristalle identifiziert werden.

Zur geeigneten Einstellung des Ausdehnungskoeffizienten und der Sintertemperatur wurde diese Apatit-Glaskeramik (A) mit den erfindungsgemäßen Alkali-Silicat-Gläsern 2 und 3 in Form von Pulvern einer mittleren Korngröße von weniger als 90 µm und in einem Gew.-Verhältnis von 30 % Apatit-Glaskeramik (A), 35 % Alkali-Silicat-Glas gemäß Beispiel 2 und 35 % Alkali-Silicat-Glas gemäß Beispiel 3 gemischt.

Diese Mischung wurde zu einem stäbchenförmigen Grünkörper in einem Vakuumofen bei einer Aufheizgeschwindigkeit von 60 °C/min und einer Haltezeit von 1 min bei 880 °C gesintert. Für die so erhaltene Probe wurde ein thermischer Ausdehnungskoeffizient von 9,5 x 10⁻⁶K⁻¹, gemessen im Temperaturbereich von 100 °C bis 400 °C, bestimmt.

Damit konnte diese Mischung zum Aufsintern auf ein Substrat mit einem thermischen Ausdehnungskoeffizienten von 10,6 x 10⁻⁶K⁻¹, wie eine Lithiumdisilicat-Glaskeramik, bei einer vorteilhaften Verarbeitungstemperatur von 830 °C verwendet werden.

Die Verarbeitung auf einem zahntechnischen Substrat kann in der Regel bei Temperaturen durchgeführt werden, die 50 °C bis 100 °C tiefer sind als die Brenntemperatur auf Quarz.

### Beispiel 8

Analog zu Beispiel 7 können zur Erzielung von gewünschten Ausdehnungskoeffizienten und Sintertemperatur auch unterschiedliche erfindungsgemäße Gläser miteinander oder mit anderen Glaskeramiken gemischt werden.

So wurde eine Pulvermischung aus 25 Gew.-% Alkali-Silicat-Glas gemäß Beispiel 3 mit 50 Gew.-% der Apatit-Glaskeramik (B) (Wärmebehandlung 1100 °C) sowie 25 Gew.-% der Apatit-Glaskeramik (A) gemäß Beispiel 7 (Wärmebehandlung 1020 °C) hergestellt, um ein erfindungsgemäßes Dentalmaterial mit einer niedrigen Sintertemperatur von 830 °C und einem Ausdehnungskoeffizienten von 9,5 x 10⁻⁶K⁻¹ zu erhalten. Ein solches Material besaß hervorragende optische Eigenschaften und eignete sich ausgezeichnet als Aufbrennkeramik für eine vollkeramische Gerüststruktur mit niedrigem Ausdehnungskoeffizienten.

Die dabei verwendete Apatit-Glaskeramik (B) hatte die Zusammensetzung SiO₂ 59,2 Gew.-%, Al₂O₃ 7,9 Gew.-%, P₂O₅ 3,0 Gew.-%, CaO 5,1 Gew.-%, F 0,6 Gew.-%, K₂O 6,8 Gew.-%, Na₂O 9,6 Gew.-%, Li₂O 0,3 Gew.-%, B₂O₃ 1,0 Gew.-%, TiO₂ 1,5 Gew.-%, ZrO₂ 2,5 Gew.-%, CeO₂ 0,5 Gew.-% und ZnO 2,0 Gew.-%. Zu ihrer Herstellung wurde ein Ausgangsglas entsprechender Zusammensetzung erschmolzen, gefrittet und zu einem Pulver aufgemahlen. Dieses Pulver wurde dann bei 1100 °C wärmebehandelt, um die Glaskeramik zu bilden.

### Beispiele 9 bis 12

In diesen Beispielen wurden weitere Mischungen von erfindungsgemäßen Alkali-Silicat-Gläsern mit Apatit-Glaskeramiken untersucht, die sich ausgezeichnet als Beschichtungs- oder Verblendmaterialien eignen, die auf Substrate mit niedrigen Ausdehnugskoefizienten aufgesintert werden können.

Es wurden die folgenden Apatit-Glaskeramiken eingesetzt:
1. Apatit-Glaskeramik (B) gemäß Beispiel 8
2. Apatit-Glaskeramik (C) der Zusammensetzung:
   SiO₂ 62,8 Gew.-%, Al₂O₃ 13,1 Gew.-%, P₂O₅ 1,2 Gew.-%, CaO 2,7 Gew.-%, F 0,6 Gew.-%, K₂O 6,3 Gew.-%, Na₂O 5,9 Gew.-%, ZrO₂ 1,7 Gew.-%, CeO₂ 0,5 Gew.-%, BaO 1,8 Gew.-%, und ZnO 3,4 Gew.-%
3. Apatit-Glaskeramik (D) der Zusammensetzung:
   SiO₂ 64,5 Gew.-%, Al₂O₃ 8,4 Gew.-%, P₂O₅ 1,1 Gew.-%, CaO 2,8 Gew.-%, F 0,7 Gew.-%, K₂O 6,6 Gew.-%, Na₂O 9,6 Gew.-%, B₂O₃ 2,2 Gew.-%, TiO₂ 1,2 Gew.-%, ZrO₂ 0,4 Gew.-% und ZnO 2,5 Gew.-%.

Die Zusammensetzungen der einzelnen Mischungen sowie die durchgeführte thermische Behandlung zur Herstellung der jeweils eingesetzten Apatit-Glaskeramik sind in Tabelle III aufgeführt.

Die für diese Mischungen bestimmten Eigenschaften sind ebenfalls in Tabelle III wiedergegeben, und sie zeigen, daß durch geeignete Wahl der Komponenten Dentalmaterialien mit an den jeweiligen Anwendungszweck angepaßten Eigenschaften erhalten werden können.

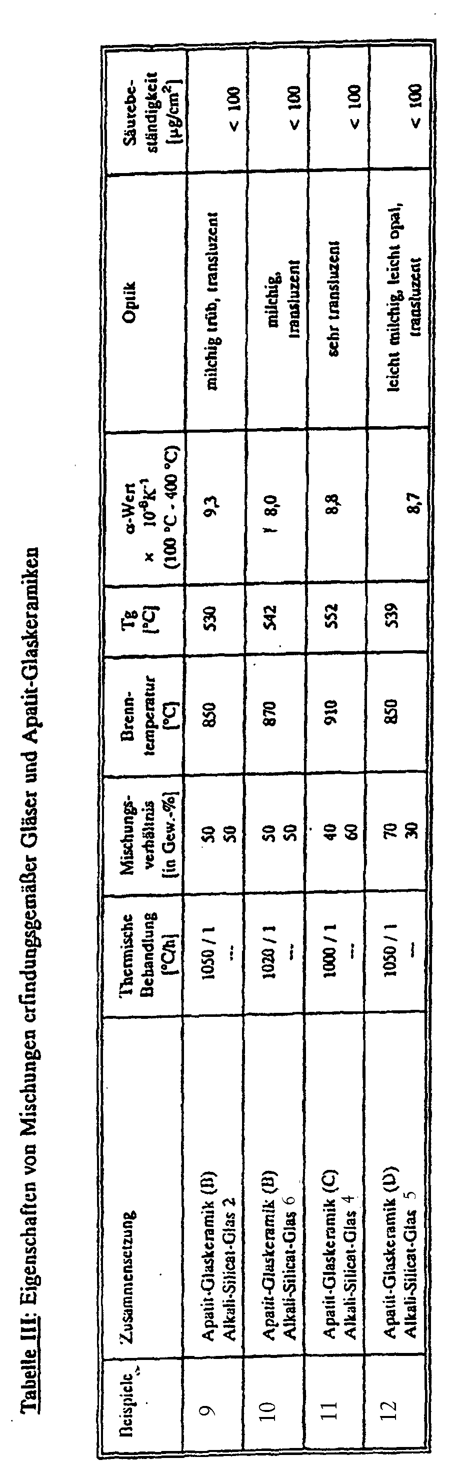

## Patentansprüche

1. Alkali-Silicat-Glas, **dadurch gekennzeichnet, daß** es die folgenden Komponenten enthält:
| Komponente | Gew.-% |
|---|---|
| SiO₂ | 55,0 bis 71,0 |
| Al₂O₃ | 5,0 bis 16,0 |
| B₂O₃ | 0,2 bis 10,0 |
| K₂O | 4,5 bis 10,0 |
| Na₂O | 3,0 bis 14,0. |
| CaO | 0,5 bis 3,0 |
| F | 0,2 bis 2,0. |

2. Glas nach Anspruch 1, **dadurch gekennzeichnet, daß** es zusätzlich mindestens eine der folgenden Komponenten enthält:
| Komponente | Gew.-% |
|---|---|
| P₂O₅ | 0 bis 0,6 |
| Li₂O | 0 bis 4,0 |
| BaO | 0 bis 5,0 |
| ZnO | 0 bis 4,0 |
| TiO₂ + ZrO₂ | 0,2 bis 5,0 |
| CeO₂ | 0 bis 2,0. |

3. Glas nach Anspruch 1 oder 2, **dadurch gekennzeichnet, daß** die Mengen einiger Komponenten unabhängig voneinander wie folgt sind:
| Komponente | Gew.-% |
|---|---|
| SiO₂ | 60,0 bis 65,0 |
| Al₂O₃ | 6,0 bis 10,0 |
| B₂O₃ | 0,5 bis 8,1 |
| K₂O | 5,5 bis 9,0 |
| Na₂O | 3,5 bis 10,0 |

4. Glas nach einem der Ansprüche 1 bis 3, **dadurch gekennzeichnet, daß** die Mengen einiger Komponenten unabhängig voneinander wie folgt sind:
| Komponente | Gew.-% |
|---|---|
| SiO₂ | 61,0 bis 64,0 |
| Al₂O₃ | 7,0 bis 9,0 |
| B₂O₃ | 0,5 bis 4,0 |
| Na₂O | 7,0 bis 9,0 |
| CaO | 0,5 bis 1,5 |
| F | 1,0 bis 2,0 |
| Li₂O | 0 bis 3,0 |
| BaO | 1,5 bis 3,5 |
| ZnO | 2,0 bis 3,5. |

5. Glas nach einem der Ansprüche 1 bis 4, **dadurch gekennzeichnet, daß** es bei thermischer Behandlung im Bereich von 600 °C bis 1000 °C für 1 Minute bis 2 Stunden nicht kristallisiert.

6. Glas nach einem der Ansprüche 1 bis 5, **dadurch gekennzeichnet, daß** es eine Sintertemperatur von 650 °C bis 1150 °C, insbesondere von 700 °C bis 1050 °C, aufweist.

7. Dentalmaterial, **dadurch gekennzeichnet, daß** es das Glas gemäß einem der Ansprüche 1 bis 6 enthält.

8. Dentalmaterial nach Anspruch 7, **dadurch gekennzeichnet, daß** es zusätzlich eine Apatit-Glaskeramik enthält.

9. Dentalmaterial nach Anspruch 8, **dadurch gekennzeichnet, daß** die Apatit-Glaskeramik CaO, P₂O₅ und F in einem Mol-Verhältnis von
| | |
|---|---|
| CaO : P₂O₅ : F | 1 : 0,020 bis 1,5 : 0,03 bis 4,2 |
und als Hauptkristallphase Apatit-Kristalle enthält.

10. Dentalmaterial nach Anspruch 8 oder 9, **dadurch gekennzeichnet, daß** die Apatit-Glaskeramik mindestens eine der folgenden Komponenten
| Komponente | Gew.-% |
|---|---|
| SiO₂ | 45,0 bis 70,0 |
| Al₂O₃ | 5,0 bis 22,0 |
| P₂O₅ | 0,5 bis 6,5 |
| K₂O | 3,0 bis 8,5 |
| Na₂O | 4,0 bis 13,0 |
| CaO | 1,5 bis 11,0 |
| F | 0,1 bis 2,5. |
und als Hauptkristallphase Apatit-Kristalle enthält.

11. Dentalmaterial nach Anspruch 10, **dadurch gekennzeichnet, daß** die Apatit-Glaskeramik zusätzlich mindestens eine der folgenden Komponenten enthält:
| Komponente | Gew.-% |
|---|---|
| B₂O₃ | 0 bis 8,0 |
| La₂O₃ | 0 bis 5,0 |
| Li₂O | 0 bis 5,0 |
| BaO | 0 bis 5,0 |
| MgO | 0 bis 5,0 |
| ZnO | 0 bis 5,0 |
| SrO | 0 bis 7,0 |
| TiO₂ | 0 bis 4,0 |
| ZrO₂ | 0 bis 4,0 |
| CeO₂ | 0 bis 3,0. |

12. Dentalmaterial nach einem der Ansprüche 7 bis 11, **dadurch gekennzeichnet, daß** es einen linearen thermischen Ausdehnungskoeffizienten von 5,5 bis 12,5 x 10⁻⁶ K⁻¹, gemessen im Bereich von 100 °C bis 400 °C, hat.

13. Verwendung des Dentalmaterials gemäß einem der Ansprüche 7 bis 12 zur Beschichtung eines Substrates und insbesondere einer dentalen Restauration.

14. Verwendung nach Anspruch 13, wobei ein Substrat auf Basis von keramischem oder glaskeramischem Werkstoff, insbesondere Lithiumdisilicat-Glaskeramik, eingesetzt wird.

15. Verwendung nach Anspruch 14, wobei die Lithiumdisilicat-Glaskeramik die folgenden Komponenten enthält:
| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0 |
| ZnO | 0 bis 8,0 |
| K₂O | 0 bis 13,5 |
| Li₂O | 11,0 bis 19,0 |
| P₂O₅ | 0 bis 11,0 |
wobei
| | |
|---|---|
| (a) Al₂O₃ + La₂O₃ | 0,1 bis 7,0 Gew.-% und |
| (b) MgO + ZnO | 0,1 bis 9,0 Gew.-% |
ausmachen.

16. Verwendung nach einem der Ansprüche 13 bis 15, wobei das Dentalmaterial auf das Substrat aufgebracht und bei Temperaturen von 650 °C bis 1150 °C gesintert wird.

17. Geformtes Dentalprodukt, **dadurch gekennzeichnet, daß** es das Alkali-Silicat-Glas gemäß einem der Ansprüche 1 bis 6 oder das Dentalmaterial gemäß einem der Ansprüche 7 bis 12 enthält.

18. Geformtes Dentalprodukt nach Anspruch 17, **dadurch gekennzeichnet, daß** es eine dentale Restauration ist.

19. Geformtes Dentalprodukt nach Anspruch 17 oder 18, **dadurch gekennzeichnet, daß** es einen Kern auf Basis von keramischem oder glaskeramischem Werkstoff und eine darauf aufgebrachte Beschichtung mit Gehalt an dem Alkali-Silicat-Glas aufweist.

20. Geformtes Dentalprodukt nach Anspruch 19, **dadurch gekennzeichnet, daß** der glaskeramische Werkstoff eine Lithiumdisilicat-Glaskeramik ist.

## Claims

1. Alkali silicate glass comprising the following components:
| Component | Wt. % |
|---|---|
| SiO₂ | 55.0 to 71.0 |
| Al₂O₃ | 5.0 to 16.0 |
| B₂O₃ | 0.2 to 10.0 |
| K₂O | 4.5 to 10.0 |
| Na₂O | 3.0 to 14.0 |
| CaO | 0.5 to 3.0 |
| F | 0.2 to 2.0 |

2. Glass according to Claim 1, **characterised in that** it additionally comprises at least one of the following components:
| Component | Wt.% |
|---|---|
| P₂O₅ | 0 to 0.6 |
| Li₂O | 0 to 4.0 |
| BaO | 0 to 5.0 |
| ZnO | 0 to 4.0 |
| TiO₂ + ZrO₂ | 0.2 to 5.0 |
| CeO₂ | 0 to 2.0 |

3. Glass according to Claim 1 or 2, **characterised in that** the concentrations of some components, independently of one another, are as follows:
| Component | Wt. % |
|---|---|
| SiO₂ | 60.0 to 65.0 |
| Al₂O₃ | 6.0 to 10.0 |
| B₂O₃ | 0.5 to 8.1 |
| K₂O | 5.5 to 9.0 |
| Na₂O | 3.5 to 10.0 |

4. Glass according to one of Claims 1 to 3, wherein the concentrations of some components, independently of one another, are as follows:
| Component | Wt.% |
|---|---|
| SiO₂ | 61.0 to 64.0 |
| Al₂O₃ | 7.0 to 9.0 |
| B₂O₃ | 0.5 to 4.0 |
| Na₂O | 7.0 to 9.0 |
| CaO | 0.5 to 1.5 |
| F | 1.0 to 2.0 |
| Li₂O | 0 to 3.0 |
| BaO | 1.5 to 3.5 |
| ZnO | 2.0 to 3.5 |

5. Glass according to one of Claims 1 to 4, **characterised in that** it does not crystallise during a heat treatment of one minute to 2 hours in the temperature range 600°C to 1000°C.

6. Glass according to one of Claims 1 to 5, **characterised in that** it has a sintering temperature of 650°C to 1150°C, particularly 700°C to 1050°C.

7. Dental material comprising the glass according to one of Claims 1 to 6.

8. Dental material according to Claim 7, which additionally comprises an apatite glass ceramic.

9. Dental material according to Claim 8, wherein the apatite glass ceramic comprises CaO, P₂O₅ and F in a molar ratio of
| | |
|---|---|
| CaO : P₂O₅ : F | 1 : 0.020 to 1.5 : 0.03 to 4.2 |
and comprises apatite crystals as the main crystal phase.

10. Dental material according to Claim 8 or 9, wherein the apatite glass ceramic comprises at least one of the following components
| Component | Wt.% |
|---|---|
| SiO₂ | 45.0 to 70.0 |
| Al₂O₃ | 5.0 to 22.0 |
| P₂O₅ | 0.5 to 6.5 |
| K₂O | 3.0 to 8.5 |
| Na₂O | 4.0 to 13.0 |
| CaO | 1.5 to 11.0 |
| F | 0.1 to 2.5 |
and comprises apatite crystals as the main crystal phase.

11. Dental material according to Claim 10, wherein the apatite glass ceramic additionally comprises at least one of the following components:
| Component | Wt.% |
|---|---|
| B₂O₃ | 0 to 8.0 |
| La₂O₃ | 0 to 5.0 |
| Li₂O | 0 to 5.0 |
| BaO | 0 to 5.0 |
| MgO | 0 to 5.0 |
| ZnO | 0 to 5.0 |
| SrO | 0 to 7.0 |
| TiO₂ | 0 to 4.0 |
| ZrO₂ | 0 to 4.0 |
| CeO₂ | 0 to 3.0 |

12. Dental material according to one of Claims 7 to 11, **characterised in that** it has a linear thermal expansion coefficient of 5.5 to 12.5 x 10⁻⁶K⁻¹, measured in the temperature range 100 to 400°C.

13. Use of the dental material according to one of Claims 7 to 12 for coating a substrate and in particular a dental restoration.

14. Use according to Claim 13, wherein a substrate based on ceramic or glass ceramic material, particularly lithium disilicate glass ceramic, is employed.

15. Use according to Claim 14, wherein the lithium disilicate glass ceramic comprises the following components:
| Component | Wt.% |
|---|---|
| SiO₂ | 57.0 to 80.0 |
| Al₂O₃ | 0 to 5.0 |
| La₂O₃ | 0.1 to 6.0 |
| MgO | 0 to 5.0 |
| ZnO | 0 to 8.0 |
| K₂O | 0 to 13.5 |
| Li₂O | 11.0 to 19.0 |
| P₂O₅ | 0 to 11.0 |
with the proviso that
| | |
|---|---|
| (a) Al₂O₃ + La₂O₃ | is 0.1 to 7.0 wt.% and |
| (b) MgO + ZnO | is 0.1 to 9.0 wt.%. |

16. Use according to one of Claims 13 to 15, wherein the dental material is applied to the substrate and sintered at temperatures of 650°C to 1150°C.

17. A shaped dental product comprising the alkali silicate glass according to one of Claims 1 to 6 or the dental material according to one of Claims 7 to 12.

18. Shaped dental product according to Claim 17, **characterised in that** it is a dental restoration.

19. Shaped dental product according to Claim 17 or 18, **characterised in that** it has a core based on ceramic or glass ceramic material and a coating applied thereto comprising the alkali silicate glass.

20. Shaped dental product according to Claim 19, wherein the glass ceramic material is a lithium disilicate glass ceramic.

## Revendications

1. Verre au silicate alcalin, **caractérisé en ce qu'**il contient les composants suivants :
| Composants | % en poids |
|---|---|
| SiO₂ | 55,0 à 71,00 |
| Al₂O₃ | 5,0 à 16,0 |
| B₂O₃ | 0,2 à 10,0 |
| K₂O | 4,5 à 10,0 |
| Na₂O | 3,0 à 14,0 |
| CaO | 0,5 à 3,0 |
| F | 0,2 à 2,0. |

2. Verre selon la revendication 1, **caractérisé en ce qu'**il contient en outre au moins un des composants suivants :
| Composants | % en poids |
|---|---|
| P₂O₅ | 0 à 0,6 |
| Li₂O | 0 à 4,0 |
| BaO | 0 à 5,0 |
| ZnO | 0 à 4,0 |
| TiO₂ + ZrO₂ | 0,2 à 5,0 |
| CeO₂ | 0 à 0,2 |

3. Verre selon la revendication 1 ou 2, **caractérisé en ce que** les quantités de certains composants sont, indépendamment les unes des autres :
| Composants | % en poids |
|---|---|
| SiO₂ | 60,0 à 65,0 |
| Al₂O₃ | 6,0 à 10,0 |
| B₂O₃ | 0,5 à 8,1 |
| K₂O | 5,5 à 9,0 |
| Na₂O | 3,5 à 10 |

4. Verre selon l'une quelconque des revendications 1 à 3, **caractérisé en ce que** les quantités de certains composants sont indépendantes les unes des autres :
| Composants | % en poids |
|---|---|
| SiO₂ | 61,00 à 64,00 |
| Al₂O₃ | 7,0 à 9,0 |
| B₂O₃ | 0,5 à 4,0 |
| Na₂O | 7,0 à 9,0 |
| CaO | 0,5 à 1,5 |
| F | 1,0 à 2,0 |
| Li₂O | 0 à 3,0 |
| BaO | 1,5 à 3,5 |
| ZnO | 2,0 à 3,5 |

5. Verre selon l'une quelconque des revendications 1 à 4, **caractérisé en ce qu'**il n'est pas cristallisé d'un traitement thermique dans la plage comprise entre 600° C et 1 000° C pendant une durée de 1 minute à 2 heures.

6. Verre selon l'une quelconque des revendications 1 à 5, **caractérisé en ce qu'**il présente une température de frittage comprise entre 650° C et 1 150° C, en particulier comprise entre 700° C et 1 050° C.

7. Matériau dentaire, **caractérisé en ce qu'**il contient le verre selon l'une quelconque des revendications 1 à 6.

8. Matériau dentaire selon la revendication 7, **caractérisé en ce qu'**il contient en outre une vitrocéramique à base d'apatite.

9. Matériau dentaire selon la revendication 8, **caractérisé en ce que** la vitrocéramique à base d'apatite contient les éléments CaO, P₂O₅ et F avec le rapport molaire suivant :
| | |
|---|---|
| CaO / P₂O₅ / F | 1 / 0,020 à 1,5 / 0,03 à 4,2 |
et **en ce qu'**il contient des cristaux d'apatite en tant que phase cristalline principale.

10. Matériau dentaire selon la revendication 8 ou 9, **caractérisé en ce que** la vitrocéramique à base d'apatite contient au moins l'un des composants suivants :
| Composants | % en poids |
|---|---|
| SiO₂ | 45,0 à 70,0 |
| Al₂O₃ | 5,0 à 22,0 |
| P₂O₅ | 0,5 à 6,5 |
| K₂O | 3,0 à 8,5 |
| Na₂O | 4,0 à 13,0 |
| CaO | 1,5 à 11,0 |
| F | 0,1 à 2,5 |
Et **en ce qu'**il contient des cristaux d'apatite en tant que phase cristalline principale.

11. Matériau dentaire selon la revendication 10, **caractérisé en ce que** la vitrocéramique à base d'apatite contient en outre au moins l'un des composants suivants :
| Composants | % en poids |
|---|---|
| B₂O₃ | 0 à 8,0 |
| La₂O₃ | 0 à 5,0 |
| Li₂O | 0 à 5,0 |
| BaO | 0 à 5,0 |
| MgO | 0 à 5,0 |
| ZnO | 0 à 5,0 |
| SrO | 0 à 7,0 |
| TiO₂ | 0 à 4,0 |
| ZrO₂ | 0 à 4,0 |
| CeO₂ | 0 à 3,0 |

12. Matériau dentaire selon l'une quelconque des revendications 7 à 11, **caractérisé en ce qu'**il présente un coefficient de dilatation thermique linéaire compris entre 5,5 et 12,5 x 10⁻⁶ K⁻¹, mesuré dans la plage comprise entre 100° C et 400° C.

13. Utilisation du matériau dentaire selon l'une quelconque des revendications 7 à 12, pour enduire un substrat et en particulier pour une restauration dentaire.

14. Utilisation selon la revendication 13, dans laquelle on utilise un substrat à base de substance céramique ou vitrocéramique, en particulier de la vitrocéramique à base de silicate de lithium.

15. Utilisation selon la revendication 14, dans laquelle la vitrocéramique à base de disilicate de lithium contient les composants suivants :
| | |
|---|---|
| SiO₂ | 57,0 à 80,0 |
| Al₂O₃ | 0 à 5,0 |
| La₂O₃ | 0,1 à 6,0 |
| MgO | 0 à 5,0 |
| ZnO | 0 à 8,0 |
| K₂O | 0 à 13,5 |
| Li₂O | 11,0 à 19,0 |
| P₂O₅ | 0 à 11,0 |
| | |
|---|---|
| a) Al₂O₃ + La₂O₃ | représente 0,1 à 7,0 % en poids et |
| b) MgO + ZnO | représente 0,1 à 9,0 % en poids. |

16. Utilisation selon l'une quelconque des revendications 13 à 15, dans laquelle le matériau dentaire est appliqué sur le substrat et est fritté à des températures comprises entre 650° C et 1 150° C.

17. Matériau dentaire mis en forme, **caractérisé en ce qu'**il contient la vitrocéramique alcaline selon l'une quelconque des revendications 1 à 6 ou le matériau dentaire selon l'une quelconque des revendications 7 à 12.

18. Matériau dentaire mis en forme selon la revendication 17, **caractérisé en ce qu'**il s'agit d'une restauration dentaire.

19. Produit dentaire mis en forme selon la revendication 17 ou 18, **caractérisé en ce qu'**il présente une partie centrale à base de substance céramique ou vitrocéramique et une enduction contenant du verre au silicate alcalin.

20. Produit dentaire mis en forme selon la revendication 19, **caractérisé en ce que** la substance vitrocéramique est une vitrocéramique à base de disilicate de lithium.
